# EUROPEAN PATENT SPECIFICATION

(11) **EP 1 831 230 B1**
(45) Date of publication and mention of the grant of the patent: **03.12.2008**
(21) Application number: 05818073.8
(22) Date of filing: 15.12.2005
(51) Int. Cl.: C07D 498/06, A61K 31/5383, A61P 31/04

(54) **PROCESS FOR OBTAINING LEVOFLOXACIN FREE FROM SALTS**
VERFAHREN ZUR GEWINNUNG VON SALZFREIEM LEVOFLOXACIN
PROCEDE DESTINE A OBTENIR DE LA LEVOFLOXACINE EXEMPTE DE SELS

(30) Priority: 31.12.2004 ES 200403166
(43) Date of publication of application: 12.09.2007
(73) Proprietor: Quimica Sintetica, S.A., 08028 Barcelona (ES)
(72) Inventor: PALOMO NICOLAU, Francisco, E-28804 Alcalà de Henares (ES); COSME GOMEZ, Antonio, E-28813 Torres de la Alameda (ES); MARTIN SANCHEZ, Rafael, E-28810 Villalbilla (ES)
(74) Representative: Ponti Sales, Adelaida
(86) International application number: PCT/IB2005/003924
(87) International publication number: WO 2006/070275

(56) References cited:
- EP-A- 1 367 132
- EP-B- 0 444 678
- WO-A-96/04247
- H. EGAWA ET. AL.: "A new Synthesis of 7H-Pyrido[1,2,3-de][1,4]benzoxazine Derivatives Including an Antibacterial Agent, Ofloxacin" CHEMICAL AND PHARMACEUTICAL BULLETIN, vol. 34, 1986, pages 4098-4102, XP001246815 cited in the application

## Description

### Field of the invention

The present invention relates to a process for preparing levofloxacin free from salts.

### State of the prior art

Levofloxacin is the international nonproprietary name of (*S*)-9-fluoro-3-methyl-10-(4-methyl-1-piperazinyl)-7-oxo-2,3-dihydro-7H-[1,2,3-de]benzoxazine-6-carboxylic acid of formula (I), with application in medicine and known for its antimicrobial activity:

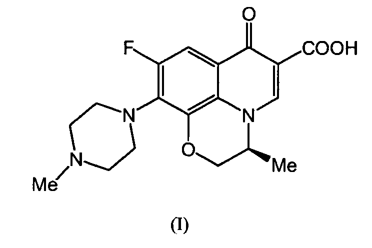

Levofloxacin crystallises in hemihydrated form or as a monohydrate, which last differs from the former in the number of molecules of water in the crystal, and in the form of anhydrous crystals produced by dehydration of these hemihydrates and monohydrates.

European patent EP 444 678 B1 discloses a process for preparing hemihydrate and monohydrate levofloxacin that substantially consists of controlling the water content of a solution of a water-miscible aqueous solvent that contains levofloxacin, such that in order to obtain the hemihydrate the water content has to be 10% or less by volume, while to obtain the monohydrate it has to have more than 10% water. Among the miscible aqueous solvents the patent mentions ethanol, methanol, propanol and acetone.

European patent application EP 1 367 132A1 discloses a process of preparing levofloxacin that consists of a total of ten steps, the last of which includes a hydrolysis of the compound (II), wherein R⁶ is a C₁-C₆ alkyl, a BZ₂ group wherein Z represents a halogen atom, a C₁-C₆ alkoxy group or a C₂-C₇ alkylcarbonyloxy group, as shown below:

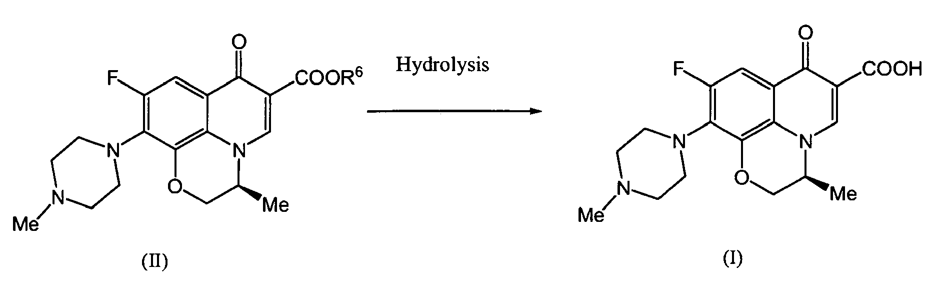

This hydrolysis step can be carried out, for example, by heating the compound (II) in the presence of a base in a protonic solvent, e.g. in the presence of trialkylamine in an alcoholic solvent. More specifically, the hydrolysis reaction can be carried out merely by heating the compound (II) in the presence of triethylamine in ethanol. This process involves a laborious purification process through to obtaining the final product. Thus, following the alkaline hydrolysis step the reaction mixture is evaporated to dryness, the residue is dissolved in dilute HCl, CHCl₃ is added and the phases are separated. The aqueous layer is adjusted to pH 11 with NaOH and then to pH 7.4 with HCl. The resulting mixture is extracted with CHCl₃. The extracts are combined and dried over sodium sulphate and the chloroform is distilled. The resulting solid is recrystallised in ethanol-ether.

In an academic paper, Hiroshi et al. in Chem. Pharm. Bull. 1986 34 (10) 4098-4102 describe a process of preparing ofloxacin (IV) whose last step comprises cyclation of the oxazine ring and subsequent alkaline hydrolysis. According to the authors, the process described is applicable to any of the optically active isomers of ofloxacin simply by using (R) or (*S*) alaninol as one of the starting products of the process. Levofloxacin is the levo-form isomer of ofloxacin.

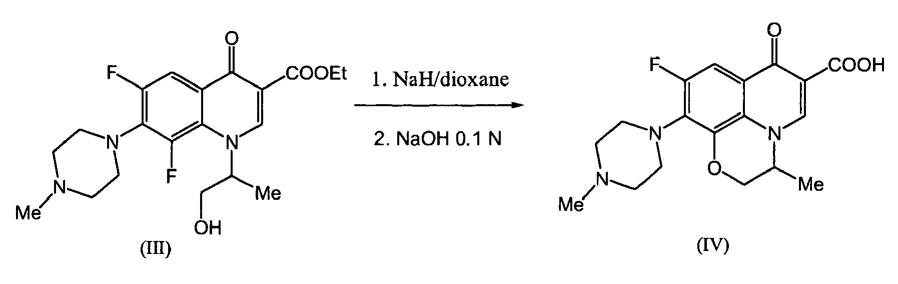

In this process the ofloxacin is isolated after a process of neutralisation with acetic acid, extraction with chloroform and recrystallisation of a chloroform-ethanol mixture.

Applied at an industrial level for manufacturing levofloxacin, these processes of alkaline hydrolysis and subsequent neutralisation involve a number of extractions in the final step. In these extractions, the organic phase has an interface that gives rise to the presence of salts and water in the product obtained. The presence of salts originates problems of crystallisation and low titrations in the solid obtained, thus rendering it necessary to reprocess the product obtained. The use of halogenated solvents also constitutes a problem from the ecological point of view, due to their highly polluting effect.

These disadvantages - complex purification process, low yields, use of halogenated solvents and eventual reprocessing - hinder and render more expensive the production of levofloxacin at industrial level.

There therefore exists a need to have a process for preparing levofloxacin free from salts with good chemical yield, without the need to carry out large numbers of extractions and substantially avoiding the use of halogenated solvents that pollute the environment.

The authors of the present invention have discovered a process for obtaining levofloxacin free from salts that does not require extractions to be carried out in the final step of purification.

### Object of the invention

The object of the present invention is a process for obtaining levofloxacin free from salts that does not require extractions be carried out in the final step of purification.

### Detailed description of the invention

The process object of the invention includes the hydrolysis of a compound (V), in the presence of a base, 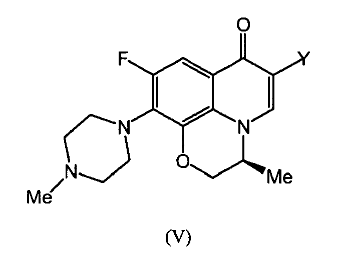 in which Y is either a COOR₁ group in which R₁ is a C₁-C₆ alkyl, or a BZ₂ group in which Z represents a halogen atom, a C₁-C₆ alkoxy group or a C₂-C₇ alkylcarbonyloxy group, or Y is a nitrile group within a water-(C₁-C₄) alkyl alcohol mixture, subsequent neutralisation, separation of the salts, followed by isolation of levofloxacin, without need to carry out any final step of extraction and without using halogenated solvents.

The process is characterised in that all the steps are carried out in the water- (C₁-C₄)alcohol mixture and in that after the neutralising agent has been added said water-(C₁-C₄)alcohol mixture must comprise proportions between 13.5 and 16% of water expressed in (w/w) or 12.2 and 14.7% of water expressed in (v/v).

The compound (V) can be prepared, for example, according to the process described in the academic publication by Hiroshi et al.

The hydrolysis reaction can be carried out by heating the product (V) in the presence of a base, such as sodium hydroxide or potassium hydroxide or any other organic or inorganic base. The C₁-C₄ alkyl alcohol can be selected from the group of compounds such as methanol, ethanol, isopropanol, 1-propanol, 1-butanol, isobutanol, sec-butanol and tert-butanol. Preferably, the C₁-C₄ alcohol is ethanol.

The neutralising agents can be selected from proton acids such as hydrochloric acid, acetic acid, sulphuric acid, phosphoric acid and nitric acid, or an ionic-exchange resin such as Dowex HCR-S Resin can be used, too. Preferably, the neutralising agent used is sulphuric acid.

The C₁-C₄ alkyl alcohol can be selected from the group of compounds such as methanol, ethanol, isopropanol, 1-propanol, 1-butanol, sec-butanol and tert-butanol. Preferably, the C₁-C₄ alcohol is ethanol.

The compound (V) reacts with the alkaline agent at a temperature between 70 and 80° C, within the water-(C₁-C₄)alcohol mixture for at least 1 hour.

The reaction is carried out in 10-15 volumes, preferably in 11-12 volumes of an alcohol/water mixture in relation to the product (V), using between 1 and 3, preferably between 1 and 2 equivalents, and more preferably 1.10 equivalents of the base.

Once the reaction has finished, the product is treated with active carbon, is filtered and a neutralising agent is added. The neutralising agent is added in a proportion of equivalent to equivalent in relation to the added base. The salts are separated, for example, by filtration at a temperature between 70 and 75° C, and the product (I) precipitates by cooling down to 0-5° C.

After adding the neutralising agent, the water-(C₁-C₄)alcohol mixture must comprise proportions of 13.5 to 16% of water expressed in (w/w) or 12.2 and 14.7% of water expressed in (v/v).

The precipitate obtained is levofloxacin (I), which is filtered, washed with the cold C₁-C₄ alcohol and dried at 40° C to constant weight.

The crude levofloxacin so obtained can later be recrystallised in an ethanol-water mixture. For example, the water content in the mixture can range between 13.5 and 16% expressed in (w/w) or 12.2 and 14.7% expressed in (v/v), preferably between 14.8 and 16% expressed in (w/w) or 13.5 and 14.7% expressed in (v/v), such that levofloxacin hemihydrate is obtained.

Surprisingly, it has been found that the new process permits levofloxacin to be obtained with a yield exceeding 70% on the basis of compound (V), without need to carry out any final step of extraction and without using halogenated solvents, with a purity and quality suitable for the preparation of pharmaceutical formulations and with a salt content not exceeding 0.1%.

The content of salts in levofloxacin is determined by the "Residue on Ignition" process of United States Pharmacopeia USP 27, equivalent to the "Sulphated Ash" process of the European Pharmacopoeia, 4th edition.

The examples that follow are outlined solely for the purposes of providing a skill in the art with a detailed explanation of the process object of the invention.

### Example 1:

50 g (0.1278 moles, 1 equivalent) of compound V (Y = COOEt) are placed in a flask, stirred into a mixture of 500 ml of ethyl alcohol, 82 ml of water and 5.5 g (0.137 moles) of sodium hydroxide. This is heated at reflux for 1 hour, cooled, treated with active carbon and filtered, washing the cake with 50 ml of ethyl alcohol. The temperature is adjusted to 50-60 °C and 6.7 g (0.068 moles) of pure sulphuric acid are added. The mixture is heated to 70-75 °C and the insoluble salts formed are removed by filtration, washing the cake with 50 ml of ethyl alcohol. The water content of the mixture must then range between 13.5 and 16% w/w (12.2 and 14.7% v/v). The filtrate obtained is cooled down to 0-5 °C and the precipitate obtained is washed with ethyl alcohol and dried, to give 35-36 g of pure levofloxacin hemihydrate. The process yield is 75%. The content in salts is lower than 0.10%.

### Example 2:

50 g of compound V (Y = COOEt) (0.1278 moles) are placed in a flask and stirred into a mixture of 500 ml of ethyl alcohol, 82 ml of water and 5.5 g (0.137 moles) of sodium hydroxide. This is heated at reflux for 1 hour, cooled, treated with active carbon and filtered, washing the cake with 50 ml of ethyl alcohol. 8.2 g (0.137 moles) of pure acetic acid are added. The water content of the mixture must then range between 13.5 and 16% w/w (12.2 and 14.7% v/v). The precipitate obtained is cooled down to 0-5 °C, filtered, washed with ethyl alcohol and dried, to give 30-31 g of pure levofloxacin hemihydrate. The process yield is 64%. The content in salts is lower than 0.10%.

### Example 3:

50 g of compound V (Y = COOEt) (0.1278 moles) is placed in a flask and stirred into a mixture of 500 ml of ethyl alcohol, 82 ml of water and 5.5 g (0.137 moles) of sodium hydroxide. This is heated at reflux for 1 hour, cooled, treated with active carbon and filtered, washing the cake with 50 ml of ethyl alcohol. 12.2 g (0.137 moles) of 65% nitric acid are added. The water content of the mixture must then range between 13.5 and 16% w/w (12.2 and 14.7% v/v). The precipitate obtained is cooled down to 0-5 °C, filtered, washed with ethyl alcohol and dried, to give 22-23 g of pure levofloxacin hemihydrate. The process yield is 47%. The content in salts is lower than 0.10%.

### Example 4:

50 g of compound V (Y = COOEt) (0.1278 moles) is placed in a flask, stirred into a mixture of 500 ml of ethyl alcohol, 50 ml of water and 5.7 g (0.14 moles) of sodium hydroxide. This is heated at reflux for 1 hour, cooled, treated with active carbon and filtered, washing the cake with 50 ml of ethyl alcohol. The temperature is adjusted to 50-60 °C and 35 g of Dowex HCR-S Resin are added. The mixture is heated to 70-75 °C and the Dowex Resin is filtered, washing the cake with 50 ml of ethyl alcohol. The water content of the mixture must then be adjusted to between 13.5 and 16% w/w (12.2 and 14.7% v/v) using the necessary amount of water. The mixture so obtained is cooled down to 0-5 °C and the precipitate obtained is washed with ethyl alcohol and dried, to give 33.5 g of pure levofloxacin hemihydrate. The process yield is 70%. The content in salts is lower than 0.10%.

## Claims

1. Process for obtaining levofloxacin containing no more than 0.1% in salts, that includes the hydrolysis of compound (V), in the presence of a base, 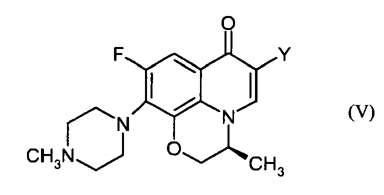 in which Y is either a COOR₁ group in which R₁ is a C₁-C₆ alkyl, or a BZ₂ group in which Z represents a halogen atom, a C₁-C₆ alkoxy group or a C₂-C₇ alkylcarbonyloxy group, or Y is a nitrile group within a water-(C₁-C₄)alcohol mixture, with subsequent neutralisation, separation of the salts, followed by the isolation of levofloxacin, without need to carry out any final step of extraction and without using halogenated solvents.

2. Process according to claim 1, **characterised in that** all the steps are carried out in the water-(C₁-C₄)alcohol mixture and **in that** after the neutralising agent has been added said water-(C₁-C₄)alcohol mixture must comprise proportions between 13.5 and 16% of water expressed in (w/w) or 12.2 and 14.7% of water expressed in (v/v).

3. Process according to claims 1 and 2, **characterised in that** the levofloxacin is isolated by precipitation of the mixture of water and a C₁-C₄ alcohol by cooling to between 0-5° C.

4. Process according to claims 1 to 3 **characterised in that** the hydrolysis reaction is carried out when the product (V) is heated in the presence of an organic or inorganic base, preferably an inorganic base, more preferably sodium hydroxide or potassium hydroxide, at a temperature between 70 and 80° C,.

5. Process according to claims 1 to 4, **characterised in that** the C₁-C₄ alcohol can be selected from the group methanol, ethanol, isopropanol, 1-propanol, 1-butanol, *sec*-butanol and *tert*-butanol, preferably ethanol.

6. Process according to claims 1 to 5, **characterised in that** the reaction is carried out in 10-15 volumes of a water- (C₁-C₄) alcohol mixture in relation to the product (V), preferably in 11-12 volumes, using between 1 and 3 equivalents of the base, preferably between 1 and 2 and more preferably still 1.10 equivalents in relation to the product (V).

7. Process according to claims 1 to 6, **characterised in that** the neutralising agent can be selected from proton acids such as hydrochloric acid, acetic acid, sulphuric acid, phosphoric acid and nitric acid, or an ionic-exchange resin such as Dowex HCR-S Resin, preferably the neutralising agent used is sulphuric acid and the neutralising agent is added in a proportion of equivalent to equivalent in relation to the added base.

8. Process according to claim 1, **characterised in that** in the compound (V) Y is equal to COOC₂H₅, the base is sodium hydroxide or potassium hydroxide and is used in a proportion of 1.10 equivalents in relation to equivalent of compound (V), the C₁-C₄ alcohol is ethanol, the water-ethanol mixture comprises proportions between 13.5 and 16% of water expressed in (w/w) or 12.2 and 14.7% of water expressed in (v/v), between 11 and 12 volumes of the water/ethanol mixture in relation to product (V) are used, the neutralising agent is sulphuric acid in a proportion of equivalent to equivalent in relation to the added base, the salts are separated by filtration at a temperature between 70 and 75° C and the hemihydrate levofloxacin product precipitates by cooling down to 0-5° C.

## Patentansprüche

1. Verfahren, um Levofloxacin zu erhalten, welches nicht mehr als 0,1% an Salzen enthält, umfassend die Hydrolyse der Verbindung (V) in Gegenwart einer Base, 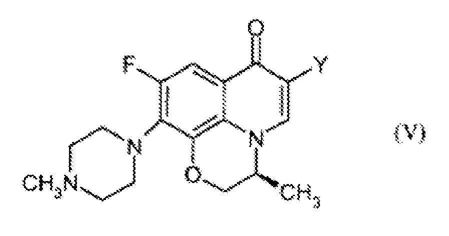 wobei Y entweder eine COOR₁-Gruppe ist, in welcher R₁ eine C₁-C₆-Alkyl- oder eine BZ₂-Gruppe ist, in der Z ein Halogenatom, eine C₁-C₆-Alkoxygruppe oder eine C₂-C₇-Alkylcarbonyloxygruppe repräsentiert oder Y eine Nitrilgruppe in einer Wasser-(C₁-C₄)Alkoholmischung ist, mit anschließender Neutralisation, Abtrennung der Salze, gefolgt von der Isolierung von Levofloxacin, ohne die Notwendigkeit, einen abschließenden Extraktionsschritt durchzuführen und ohne Verwendung halogenierter Lösungsmittel.

2. Verfahren nach Anspruch 1, **dadurch gekennzeichnet, dass** alle Schritte in der Wasser-(C₁-C₄)Alkoholmischung durchgeführt werden und dass nach Zugabe des Neutralisierungsmittels die Wasser-(C₁-C₄)Alkoholmischung Anteile von 13,5 bis 16% an Wasser, ausgedrückt in (w/w), oder 12,2 bis 14,7% Wasser, ausgedrückt in (v/v), umfassen muss.

3. Verfahren nach den Ansprüchen 1 und 2, **dadurch gekennzeichnet, dass** das Levofloxacin durch Präzipitation der Mischung aus Wasser und einem C₁-C₄-Akohol durch Abkühlen auf 0-5°C isoliert wird.

4. Verfahren nach den Ansprüchen 1 bis 3, **dadurch gekennzeichnet, dass** die Hydrolysereaktion durchgeführt wird, wenn das Produkt (V) in Gegenwart einer organischen oder anorganischen Base, bevorzugt einer anorganische Base, bevorzugter Natriumhydroxid oder Kaliumhydroxid, bei einer Temperatur von 70 bis 80°c erhitzt wird.

5. Verfahren nach den Ansprüchen 1 bis 4, **dadurch gekennzeichnet, dass** der C₁-C₄-Alkohol auswählbar ist aus der Gruppe von Methanol, Ethanol, Isopropanol, 1-Propanol, 1-Butanol, sec-Butanol und tert-Butanol, bevorzugt Ethanol.

6. Verfahren nach einem der Ansprüche 1 bis 5, **dadurch gekennzeichnet, dass** die Reaktion in 10-15 Volumen einer Wasser-(C₁-C₄)Alkoholmischung, bezogen auf das Produkt (V), bevorzugt in 11-12 Volumen, durchgeführt wird, unter Verwendung von 1 bis 3 Äquivalenten der Base, bevorzugt 1 bis 2 und bevorzugter 1,10 Äquivalenten, bezogen auf das Produkt (V).

7. Verfahren nach den Ansprüchen 1 bis 6, **dadurch gekennzeichnet, dass** das Neutralisationsmittel auswählbar ist aus Protonsäuren, wie Salzsäure, Essigsäure, Schwefelsäure, Phosphorsäure und Salpetersäure, oder einem Ionenaustauschharz, wie Dowex HCR-S-Harz, wobei als Neutralisationsmittel bevorzugt Schwefelsäure verwendet wird und das Neutralisationsmittel in einem Äquivalenzanteil zugegeben wird, der in Bezug zur zugegebenen Base äquivalent ist.

8. Verfahren nach Anspruch 1, **dadurch gekennzeichnet, dass** in der Verbindung (V) Y gleich COOC₂H₅ ist, die Base Natriumhydroxid oder Kaliumhydroxid ist und in einem Anteil von 1,10 Äquivalenten in Bezug auf das Äquivalent der Verbindung (V) eingesetzt wird, der C₁-C₄-Alkohol Ethanol ist, die Wasser-Ethanol-Mischung Anteile von 13,5 bis 16% an Wasser, ausgedrückt in (w/w), oder 12,2 bis 14,7% an Wasser, ausgedrückt in (v/v), aufweist, 11 bis 12 Volumina der Wasser/Ethanol-Mischung, in Bezug auf das Produkt (V), eingesetzt werden, das Neutralisationsmittel Schwefelsäure in einem Äquivalenzanteil ist, der in Bezug auf die zugegebene Base äquivalent ist, die Salze durch Filtration bei einer Temperatur von 70 bis 75°C abgetrennt werden und das Levofloxacin-Halbhydratprodukt durch Abkühlen auf 0-5°C präzipitiert.

## Revendications

1. Procédé pour l'obtention de lévofloxacine ne contenant pas plus de 0,1 % de sels, qui comprend l'hydrolyse d'un composé (V), en présence d'une base, 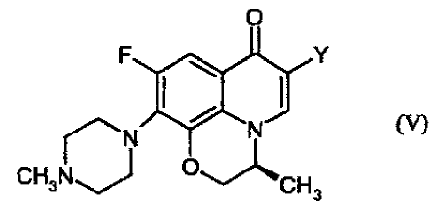 dans lequel Y est soit un groupe COOR₁ dans lequel R₁ est un groupe alkyle en C₁-C₆, ou un groupe BZ₂ dans lequel Z représente un atome d'halogène, un groupe alkoxy en C₁-C₆ ou un groupe alkylcarbonyloxy en C₂-C₇, ou Y est un groupe nitrile, dans un mélange d'eau et d'un alcool en C₁-C₄, avec une neutralisation ultérieure, la séparation des sels, puis l'isolement de la lévofloxacine, sans nécessiter l'exécution d'une quelconque étape finale d'extraction et sans employer de solvants halogénés.

2. Procédé selon la revendication 1, **caractérisé en ce que** toutes les étapes sont effectuées dans le mélange d'eau et d'un alcool en C₁-C₄, et **en ce qu'**après l'ajout de l'agent de neutralisation, ledit mélange d'eau et d'un alcool en C₁-C₄ doit comprendre des proportions d'eau de 13,5 à 16 % exprimées en poids/poids, ou de 12,2 à 14,7 % exprimées en volume/volume.

3. Procédé selon les revendications 1 et 2, **caractérisé en ce que** la lévofloxacine est isolée par précipitation dans le mélange d'eau et d'un alcool en C₁-C₄, par refroidissement à 0 jusqu'à 5 °C.

4. Procédé selon les revendications 1 à 3, **caractérisé en ce que** la réaction d'hydrolyse est effectuée lorsque le produit (V) est chauffé en présence d'une base organique ou inorganique, de préférence une base inorganique, préférentiellement l'hydroxyde de sodium ou l'hydroxyde de potassium, à une température de 70 à 80 °C.

5. Procédé selon les revendications 1 à 4, **caractérisé en ce que** l'alcool en C₁-C₄ peut être choisi dans le groupe constitué du méthanol, de l'éthanol, de l'isopropanol, du 1-propanol, du 1-butanol, du sec-butanol et du tert-butanol, de préférence, de l'éthanol.

6. Procédé selon les revendications 1 à 5, **caractérisé en ce que** la réaction est effectuée dans 10 à 15 volumes d'un mélange d'eau et d'un alcool en C₁-C₄ par rapport au produit (V), de préférence, dans 11 à 12 volumes, en employant de 1 à 3 équivalents de la base, de préférence de 1 à 2 et, encore préférentiellement 1,10 équivalents par rapport au produit (V).

7. Procédé selon les revendications 1 à 6, **caractérisé en ce que** l'agent de neutralisation peut être choisi parmi les acides protiques tels que l'acide chlorhydrique, l'acide acétique, l'acide sulfurique, l'acide phosphorique et l'acide nitrique, ou une résine échangeuse d'ion telle que la résine Dowex HCR-S, l'agent de neutralisation employé étant, de préférence, l'acide sulfurique, et **en ce que** l'agent de neutralisation est ajouté en une proportion d'équivalents à équivalents par rapport à la base ajoutée.

8. Procédé selon la revendication 1, **caractérisé en ce que**, dans le composé (V), Y représente COOC₂H₅, la base est l'hydroxyde de sodium ou l'hydroxyde de potassium, et **en ce qu'**il est employé en une proportion de 1,10 équivalents par équivalent du composé (V), l'alcool en C1-C4 est l'éthanol, le mélange d'eau et d'éthanol comprend des proportions d'eau de 13,5 à 16 % exprimées en poids/poids, ou de 12,2 à 14,7 % exprimées en volume/volume, on emploie de 11 à 12 volumes du mélange d'eau et d'éthanol par rapport au produit (V), l'agent de neutralisation étant l'acide sulfurique en une proportion d'équivalents à équivalents par rapport à la base ajoutée, les sels sont séparés par filtration à une température de 70 à 75 °C, et le produit de type lévofloxacine hémihydratée précipite par refroidissement à 0-5 °C.
